Europäisches Patentamt

European Patent Office

Office européen des brevets

(19)

(11) Numéro de publication: **0 266 469 B1**

(12) **FASCICULE DE BREVET EUROPEEN**

(45) Date de publication de fascicule du brevet:
**27.12.91**

(51) Int. Cl.5: **A61M 25/00**

(21) Numéro de dépôt: **86402489.8**

(22) Date de dépôt: **07.11.86**

(54) **Sonde oesophagienne.**

(43) Date de publication de la demande:
**11.05.88 Bulletin 88/19**

(45) Mention de la délivrance du brevet:
**27.12.91 Bulletin 91/52**

(84) Etats contractants désignés:
**AT BE CH DE ES GB GR IT LI LU NL SE**

(56) Documents cités:
**EP-A- 0 088 714**
**EP-A- 0 162 982**
**US-A- 3 046 988**
**US-A- 3 742 958**
**US-A- 3 780 740**

(73) Titulaire: **SYNTHELABO**
**58 rue de la Glacière**
**F-75013 Paris(FR)**

(72) Inventeur: **Vankemmel, Jacqueline**
**277, rue du Ballon**
**F-59800 Lille(FR)**

(74) Mandataire: **Ludwig, Jacques et al**
**SYNTHELABO, Service Brevets, 22, avenue**
**Galilée, B.P. 72**
**F-92352 Le Plessis Robinson Cédex(FR)**

## Description

La présente invention a pour objet une sonde oesophagienne, prévue notamment pour les scléroses oesophagiennes en urgence.

On connaît déjà une sonde oesophagienne (type Linton-Nachlas) constituée d'un tube souple renfermant plusieurs tubulures et munie à son extrémité distale d'un ballonnet gonflable destiné à assurer l'étanchéité entre l'oesophage et l'estomac. Cette sonde comporte divers inconvénients, parmi lesquels

- la difficulté de sa mise en place, car son diamètre, et surtout celui de son ballonnet, sont importants,
- la difficulté de son maintien en place, car la traction qu'on lui applique nécessite l'utilisation d'un lit spécial muni de poulies et de contrepoids,
- l'impossibilité de procéder à des examens endoscopiques, la lumière de l'oesophage étant occupée presque en totalité par la sonde.

Une autre sonde connue (type Blakemore) comporte deux ballonnets gonflables : le ballonnet distal (gonflé dans l'estomac et amené au contact du cardia) permet le bon positionnement de la sonde , et le ballonnet proximal (occupant tout le volume de l'oesophage, ou tout au moins la partie présentant des varices) permet de comprimer ces dernières pour interrompre le saignement. Une sonde de ce type est décrite par exemple dans le brevet des Etats Unis d'Amérique numéro 3 046 988. Comme le précédent type de sonde, celle-ci ne permet pas l'examen des parties lésées (situées sous le ballonnet) et, de plus, le ballonnet de compression des varices doit être gonflé avec prudence, du fait du risque d'ischémie, voire de rupture des tissus oesophagiens rendus fragiles par les varices.

La présente invention permet de remédier aux inconvénients des sondes connues, en particulier lorsqu'il s'agit d'intervenir sur un patient qui doit être transporté en urgence vers un centre de soins, c'est-à-dire lorsque la sonde n'est en place que pour une période de temps relativement courte, de l'ordre de quelques heures.

Ainsi la sonde selon l'invention comporte une extrémité distale, une tubulure unique entourée d'un fourreau présentant une prédécoupe sur toute sa longueur, sur lequel peut coulisser une bague, un ballonnet unique et un embout proximal, et se caractérise essentiellement en ce que le fourreau présente une seconde prédécoupe circonferentielle, au voisinage de son extrémité proximale, à une distance de l'extrémité proximale sensiblement égale à la dimension du ballonnet mesurée le long de la tubulure et permettant d'en séparer un tronçon.

La description qui va suivre en regard des dessins annexés fera mieux comprendre comment est constituée la sonde selon l'invention. Il est bien entendu que ces dessins schématiques sont essentiellement destinés à faire comprendre le fonctionnement et l'utilisation de la sonde ; il ne font qu'illustrer une forme de réalisation, les dimensions et les proportions de la sonde pouvant varier sans sortir du cadre de la présente invention.

La figure 1 représente l'ensemble 1 de la sonde avant sa mise en place sur le patient.

La figure 2 représente la sonde débarassée de son fourreau 2 et 4, le ballonnet 3 étant à l'état dégonflé.

La figure 2a est une vue partielle, sous un autre angle, du haut de la figure 2.

La figure 3 représente la sonde sans le fourreau 2 et 4, le ballonnet 3 étant gonflé.

La figure 4 représente la sonde telle qu'elle peut se présenter juste après introduction dans le patient, c'est-à-dire avec le fourreau 2 en place, mais ramené vers l'extrémité proximale grâce à l'enlèvement d'un tronçon séparable 4, de manière à dégager le ballonnet 3.

La figure 5 représente la sonde après introduction dans l'oesophage du patient et après gonflage du ballonnet.

La figure 6 est une vue agrandie d'une variante de réalisation de l'extrémité distale de la sonde.

Les figures 7a, 7b et 7c sont des vues en coupe partielle de la sonde placée dans l'oesophage d'un patient.

La sonde selon l'invention présente une extrémité distale effilée 5, destinée à permettre son introduction dans l'oesophage d'un patient, soit par la voie nasale, soit par la voie buccale. Cette extrémité 5 a une forme telle qu'elle facilite à la fois le guidage de la sonde et l'élargissement du passage, en particulier lorsqu'on l'introduit par la voie nasale. Ainsi l'extrémité 5 peut se présenter avantageusement avec une première partie cylindrique 6, à bout arrondi, suivie d'une partie tronconique 7 dont le diamètre maximal correspond au diamètre du fourreau 2.

L'extrémité effilée 5 peut porter avantageusement un marquage radio-opaque (non représenté), par exemple un marquage circonférentiel au voisinage de son plus grand diamètre, ou encore un marquage longitudinal, depuis la pointe 6 jusqu'à son plus grand diamètre.

L'extrémité effilée 5 est portée par une fine tubulure souple 8, qu'elle obture à l'extrémité distale. La tubulure 8 est munie à l'extrémité proximale d'un embout 9, permettant le raccordement à un moyen de gonflage (non représenté) tel qu'une seringue, et permettant également une bonne prise ne main en vue d'exercer une traction. Au voisinage de son extrémité distale la tubulure 8 présente une perforation latérale 10 débouchant à l'intérieur d'un ballonnet 3.

Entre l'embout 9 et l'extrémité 5 la tubulure 8 est entourée d'un fourreau 2 et 4 de forme générale cylindrique et présentant sur toute sa longueur une prédécoupe 11, ou pré-fente, c'est à dire une ligne de déchirement facilité, permettant de le fendre sur sa longueur sans l'aide d'un instrument tranchant. Un tel fourreau est décrit dans la demande de brevet européen numéro 0 162 982. Le fourreau selon l'invention présente avantageusement une seconde prédécoupe 12 permettant d'en séparer un tronçon 4, également sans outil.

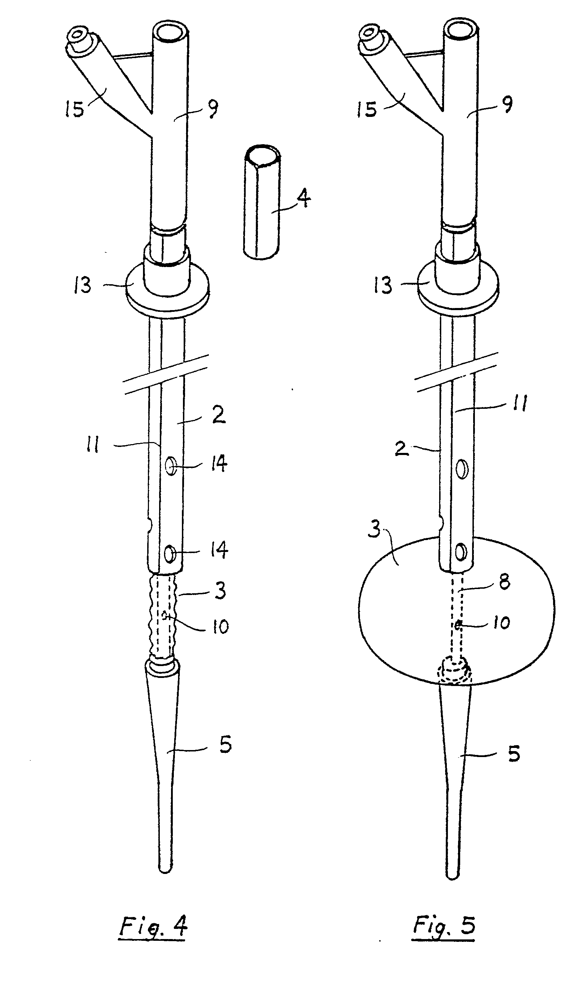

La séparation du tronçon 4 permet à la partie restante 2 du fourreau de coulisser en direction de l'embout proximal 9, de manière à dégager le ballonnet 3 entourant la tubulure 8. La longueur du tronçon 4 est donc telle qu'elle correspond sensiblement à la dimension du ballonnet 3 gonflé, mesurée le long de la tubulure 8 (figure 5).

La sonde selon l'invention comporte également une bague 13 coulissant sur le fourreau 2 et 4 à frottement dur. Lorsque la sonde est en place sur un patient, le tronçon 4 enlevé et le ballonnet 3 gonflé, la bague 13 peut être déplacée le long du fourreau 2 pour venir prendre appui, à la manière d'une butée, sur le bord de l'orifice nasal ou buccal du patient, afin de maintenir une certaine force de traction sur le ballonnet 3 par l'intermédiaire de la tubulure 8. La bague 13 peut aussi coulisser vers l'extrémité proximale pour venir entourer l'embout 9, permettant ainsi le dégagement facile du fourreau 2, ce dernier étant progressivement fendu selon sa longueur au cours de son extraction.

Enfin, bien que destinée essentiellement à une utilisation de courte durée, c'est-à-dire n'impliquant pas la nécessité d'évacuer des sécrétions telles que salive ou saignement, la sonde selon l'invention peut comporter des moyens d'évacuation consistant d'une part en des perforations 14 ménagées dans le fourreau 2 et, d'autre part, et un canal 9', 9" communiquant avec l'intérieur du fourreau 2 ou 4 en traversant l'embout 9, et pouvant soit être obturé, par exemple par un simple bouchon, soit être raccordé à un système d'aspiration (non représenté).

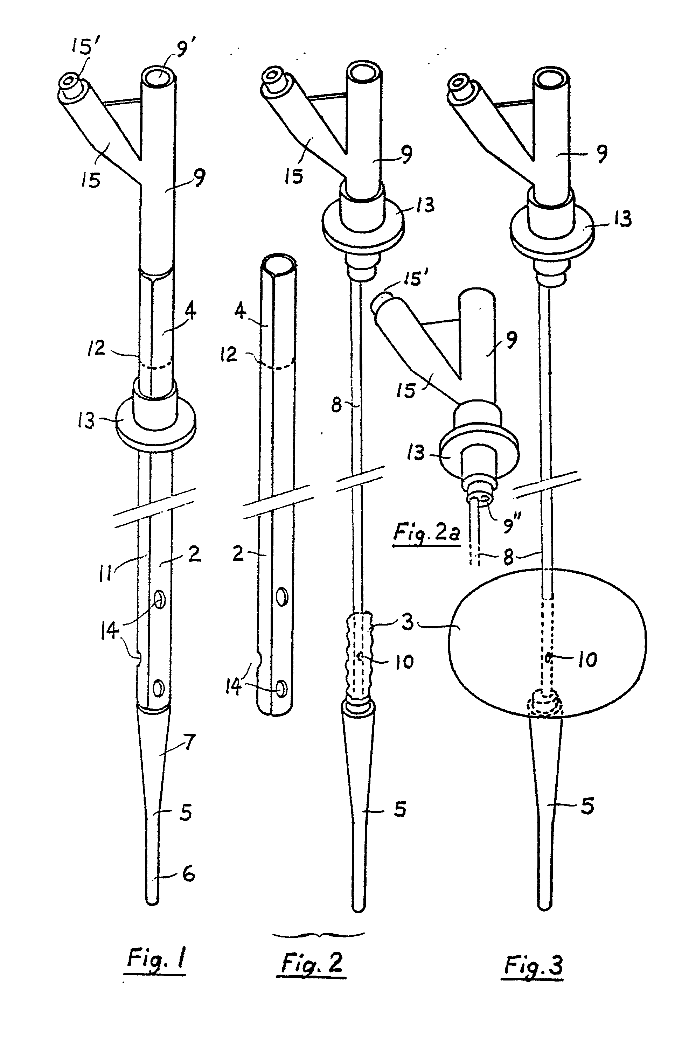

Les perforations latérales 14 sont de préférence disposées de manière telle que deux d'entre elles soient en regard du ballonnet 3, et qu'une troisième lui soit sus-jacente (figures 1 et 2).

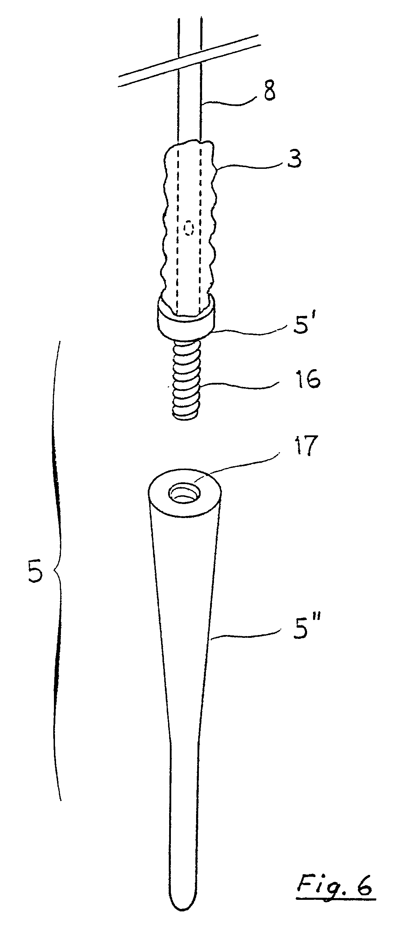

Une variante de réalisation de l'extrémité distale de la sonde selon l'invention est illustrée par la figure 6. Cette variante consiste en ce que, au lieu d'être solidaire et inamovible de l'extrémité distale de la tubulure 8, l'extrémité effilée 5 peut être détachée de la sonde.

Grâce à cette variante il devient possible, avec une sonde qui a déjà été utilisée, d'enlever l'extrémité effilée 5, d'enfiler sur la tubulure 8 un nouveau fourreau 2 et 4 entier, c'est-à-dire pré-fendu et muni du tronçon détachable 4, puis de remettre en place l'extrémité effilée 5, ce qui fournit une sonde prête à un nouvel emploi. Etant donné que le plus grand diamètre de l'extrémité effilée 5 est égal au diamètre extérieur du fourreau 2, il serait en effet impossible d'enfiler un fourreau neuf "par dessus" l'extrémité effilée 5 sans provoquer le déchirement du fourreau le long de la ligne de rupture privilégiée 11.

Un mode de réalisation d'une extrémité effilée 5 amovible peut consister par exemple en ce que cette dernière est en réalité constituée de deux tronçons dont la réunion correspond à une extrémité effilée 5 telle que représentée sur les figures 1 à 5.

Ainsi un premier tronçon 5', dont le plus grand diamètre est inférieur au diamètre intérieur du fourreau 2 et 4, vient coiffer et obturer l'extrémité distale de la tubulure 8, de manière étanche et inamovible. L'extrémité distale de ce tronçon 5' comporte un moyen d'assemblage 16, par exemple un filetage mâle, et le second tronçon 5" de l'extrémité effilée 5 comporte un moyen d'assemblage de forme conjuguée 17, par exemple un taraudage ; la réunion des deux tronçons 5' et 5" s'opère donc par vissage.

Toutefois, comme le tronçon distal 5" n'est jamais soumis à de grandes forces de traction par rapport au tronçon 5', on peut éventuellement prévoir un mode d'assemblage plus simple, par exemple un dispositif de type bouton-pression ou d'encliquetage, l'un des tronçons 5' et 5" présentant une forme mâle et l'autre une forme femelle conjuguée. Un tel type d'encliquetage est rendu possible grâce au matériau relativement souple qui constitue le tronçon 5", le tronçon 5' pouvant être en un matériau plus rigide.

Le mode d'utilisation de la sonde est le suivant :

Chez un malade porteur de varices oesophagiennes rompues, on introduit l'ensemble de la sonde 1 dans la lumière oesophagienne par voie buccale ou nasale, de façon aisée grâce à l'extrémité effilée 5. On descend cette dernière jusque dans la cavité gastrique, on procède à l'ablation du tronçon séparable 4, après avoir fait coulisser la bague 13 au delà de la prédécoupe 12, et on amène la partie restante du fourreau 2 en butée avec l'embout 9. Grâce à cette manoeuvre le ballonnet 3 se trouve libéré du fourreau 2, de sorte qu'on peut le gonfler dans la cavité gastrique, en injectant de l'air par l'embout 9, par exemple au moyen d'une seringue. On exerce alors une traction sur l'embout 9, et on fait coulisser la bague 13 le long du fourreau 2 jusqu'à l'amener en butée sur le bord de l'orifice d'introduction de la sonde, c'est-à-dire la narine ou la bouche du patient.

L'hémostase est donc assurée et le patient peut être transporté sans être relié à un dispositif externe de traction.

Lorsque le patient se trouve présent dans un centre d'endoscopie digestive, on peut alors procéder au retrait du fourreau 2. Pour cela, tout en exerçant manuellement une traction sur l'embout 9, on déplace d'abord la bague 13 en la faisant coulisser vers l'extrémité proximale, jusque sur l'embout 9, puis on retire le fourreau 2, opération rendue possible grâce à la prédécoupe longitudinale 11.

L'oesophage ne contient alors plus que la tubulure 8, qui est suffisamment fine pour permettre l'introduction d'un appareil d'endoscopie tel qu'un fibroscope avec canal opérationnel permettant la réalisation d'injections sclérosantes.

L'ablation complète de la sonde se fait évidemment après dégonflage du ballonnet 3, par simple traction sur l'embout 9.

Dans le cas où l'extrémité distale de la sonde est conformée selon la figure 6, il est possible, après ablation et lavage de la sonde, de détacher le tronçon distal 5″ de l'extrémité effilée 5, d'enfiler un fourreau 2 et 4 neuf autour de la tubulure 8 et du ballonnet 3 dégonflé, et enfin de remettre en place le tronçon distal 5″, avant de stériliser l'ensemble en vue d'une nouvelle utilisation.

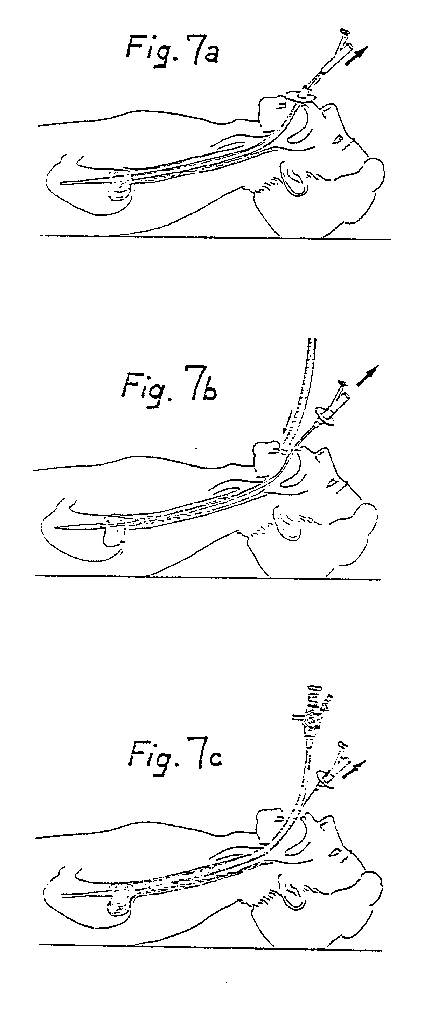

Les figures 7a, 7b et 7c illustrent respectivement

- l'utilisation à visée hémostatique de la sonde dont la traction sur le ballonnet est maintenue grâce à la bague 13 prenant appui sur l'orifice buccal ;
- l'utilisation à visée hémostatique et diagnostique : la traction manuelle sur le ballonnet est maintenue au cours de l'exploration endoscopique ;
- l'utilisation à visée hémostatique et thérapeutique : la traction sur le ballonnet contrôle l'hémostase des varices oesophagiennes qui sont l'objet d'une oblitération endoscopique.

L'exemple suivant illustre un mode de réalisation préféré de la sonde de l'invention.

Les dimensions indiquées ne constituent nullement une limitation de l'invention encore que, la sonde étant destinée essentiellement à des patients adultes, celles-ci ne peuvent varier que dans des intervalles assez restreints.

EXEMPLE.

Une sonde selon l'invention comprend une extrémité distale effilée 5 de 12 cm de long, dont une partie cylindrique 6 de 4 mm de diamètre et 6 cm de long, et une partie tronconique de 4 à 9 mm de diamètre et 6 cm de long.

Cette extrémité 5 est en une matière relativement souple, par exemple en polychlorure de vinyle. Elle porte un marquage radio-opaque sur sa périphérie, au voisinage de son plus grand diamètre. La partie intermédiaire de la sonde comporte une fine tubulure 8 centrale, résistante et inextensible, de 4 mm de diamètre, obturée à son extrémité distale par la pointe 5, mais débouchant par un orifice 10 dans un ballonnet 3 dont le volume maximal est de 250 ml environ lorsqu'il est gonflé et dont le diamètre mesuré le long de la tubulure 8 est de 45 mm. Ledit ballonnet est en polychlorure de vinyle, en latex ou en silicone. L'extrémité proximale de la tubulure 8 aboutit dans un embout rigide 15 muni d'une valve d'obturation 15′ pouvant recevoir une seringue pour le gonflage et le dégonflage du ballonnet 3. L'embout 15 est adossé latéralement à un embout principal 9, de 8 cm de long et 9 mm de diamètre, qui peut être connecté par son pavillon 9′ à un dispositif d'aspiration. Le canal d'aspiration traverse l'embout 9 et débouche en 9″ à l'intérieur du fourreau 2 ou 4, ce qui permettra, grâce aux perforations latérales 14 du fourreau 2, d'aspirer les sécrétions (salive, sang) séjournant dans la lumière oesophagienne.

Entre ledit embout 9 et l'extrémité 5, la longueur de la tubulure 8 est d'environ 50 cm. La tubulure 8 est en une matière inextensible (pour pouvoir supporter un effort de traction) et relativement rigide (pour permettre, le ballonnet 3 étant gonflé, le maintien en place de l'extrémité 5 lorsqu'on fait coulisser le fourreau 2 en direction de l'embout 9), par exemple en polychlorure de vinyle.

Un fourreau 2 et 4, de 9 mm de diamètre et 50 cm de longueur entoure la tubulure. Il présente une pré-découpe longitudinale 11 sur toute sa longueur et une pré-découpe circulaire 12 à 45 mm de son extrémité proximale (la longueur du tronçon 4 est donc égale au diamètre du ballonnet 3 mesuré le long de la tubulure 8).

Enfin la sonde comprend une bague 13 en une matière relativement rigide, dont le diamètre intérieur, de l'ordre de 9 mm, permet un coulissement à frottement dur sur le fourreau 2 et 4.

Dans le cas de la variante selon la figure 6, l'extrémité effilée 5 est constituée de deux parties 5′ et 5″. La première, solidaire de l'extrémité distale de la tubulure 8, a un diamètre maximal de 6 mm (le diamètre intérieur du fourreau 2 étant de 7 mm), et est prolongé par une partie mâle filetée 16 de 4 mm de diamètre et 10 mm de long.

La partie distale 5″ comprend une portion cylindrique de 4 mm de diamètre et une portion tronconique de 4 à 9 mm de diamètre, la longueur de l'ensemble étant de 12 cm, comme indiqué plus haut. Elle présente sur sa face plane proximale un taraudage borgne 17 pouvant recevoir par vissage la partie mâle conjuguée 16 du tronçon 5′.

## Revendications

1. Sonde oesophagienne comportant une extrémité distale (5), une tubulure unique (8) entourée d'un fourreau (2,4) présentant une prédécoupe (11) sur toute sa longueur, sur lequel peut coulisser une bague (13), un ballonnet (3) unique et un embout proximal (9), caractérisée en ce que le fourreau (2,4) présente une seconde prédécoupe, circonférentielle (12), au voisinage de son extrémité proximale, à une distance de l'extrémité proximale sensiblement égale à la dimension du ballonnet (3) mesurée le long de la tubulure (8), et permettant d'en séparer un tronçon (4).

2. Sonde selon la revendication 1, caractérisée en ce que le fourreau (2) présente des perforations latérales (14) au voisinage de son extrémité distale.

3. Sonde selon l'une quelconque des revendications 1 et 2, caractérisée en ce que le diamètre de l'embout (9), le diamètre du fourreau (2,4) et le diamètre maximal de l'extrémité (5) sont sensiblement égaux.

4. Sonde selon l'une quelconque des revendications 1 à 3, caractérisée en ce que l'extrémité distale (5) est constituée de deux tronçons 5' et 5", le premier étant solidaire et inamovible de la tubulure 8 et comportant un moyen d'assemblage (16), le second étant amovible et comportant un moyen d'assemblage (17) de forme conjuguée.

5. Sonde selon la revendication 4, caractérisée en ce que l'un des moyens d'assemblage (16, 17) est un filetage mâle et l'autre un taraudage conjugué.

6. Sonde selon la revendication 4, caractérisée en ce que les moyens d'assemblage (16, 17) sont constitués par un dispositif d'encliquetage.

7. Sonde selon l'une quelconque des revendications 1 à 6, caractérisée en ce qu'elle est munie de moyens d'aspiration (9,9',9",14).

8. Sonde selon l'une quelconque des revendications 1 à 7, caractérisée en ce que l'extrémité proximale de la tubulure (8) est reliée à un embout (15) muni d'une valve (15').

## Claims

1. Oesophageal probe comprising a distal end (5), a single tube (8) surrounded by a sheath (2,4) having a preliminary cut (11) over its entire length, on which a ring (13) can slide, a single balloon (3) and a proximal joining piece (9), characterised in that the sheath (2,4) has a second circumferential preliminary cut (12), in the vicinity of its proximal end, at a distance from the proximal end essentially equal to the dimension of the balloon (3) measured along the tube (8), and making it possible to separate a portion (4) therefrom.

2. Probe according to Claim 1, characterised in that the sheath (2) has lateral perforations (14) in the vicinity of its distal end.

3. Probe according to either one of Claims 1 and 2, characterised in that the diameter of the joining piece (9), the diameter of the sheath (2,4) and the maximum diameter of the end (5) are essentially equal.

4. Probe according to any one of Claims 1 to 3, characterised in that the distal end (5) consists of two portions 5'and 5", the first being integral with the tube 8 and non-removable therefrom and comprising an assembly means (16), the second being removable and comprising an assembly means (17) of conjugate form.

5. Probe according to Claim 4, characterised in that one of the assembly means (16, 17) is an external thread and the other a conjugate internal thread.

6. Probe according to Claim 4, characterised in that the assembly means (16, 17) consist of a snap-locking device.

7. Probe according to any one of Claims 1 to 6, characterised in that it is equipped with suction means (9, 9', 9", 14).

8. Probe according to any one of Claims 1 to 7, characterised in that the proximal end of the tube (8) is connected to a joining piece (15) equipped with a valve (15').

## Patentansprüche

1. Speiseröhrensonde mit einem jenseitigen Ende (5), mit einem einzigen Rohr (8), das von einer Hülse (2, 4) umgeben ist, die über einen VorEinschnitt (11) über ihre gesamte Länge verfügt und auf der ein Ring (13) verschiebbar ist, einem einzigen Ballon (3) und einem diesseitigen Übergangsrohr (9), dadurch **gekennzeichnet,** daß die Hülse (2, 4) einen zweiten Vor-Einschnitt (12) in Umfangsrichtung in der Nähe des diesseitigen Endes aufweist, in einem Abstand zu dem diesseitigen Ende, der im wesentlichen gleich der Abmessung des Ballons (3) in Längsrichtung des Rohres (8) ist, welcher Vor-Einschnitt das Abtrennen eines Teilabschnitts (4) gestattet.

2. Sonde nach Anspruch 1, dadurch **gekennzeichnet,** daß die Hülse (2) seitliche Durchbrechungen (14) in der Nähe des jenseitigen Endes aufweist.

3. Sonde nach einem der Ansprüche 1 und 2, dadurch **gekennzeichnet,** daß der Durchmesser des Übergangsrohres (9), der Durchmesser der Hülse (2, 4) und der größte Durchmesser des Endes (5) im wesentlichen gleich sind.

4. Sonde nach einem der Ansprüche 1 bis 3, dadurch **gekennzeichnet,** daß das jenseitige Ende (5) durch zwei Teilabschnitte (5', 5") gebildet wird, deren erster fest und unlösbar in bezug auf das Rohr (8) ist und eine Verbindungseinrichtung (16) aufweist und deren zweiter lösbar ist und eine in der Form korrespondierende Befestigungseinrichtung (17) aufweist.

5. Sonde nach Anspruch 4, dadurch **gekennzeichnet,** daß eine der Verbindungseinrichtungen (16, 17) ein Außengewinde und die andere ein entsprechendes Innengewinde ist.

6. Sonde nach Anspruch 4, dadurch **gekennzeichnet,** daß die Verbindungseinrichtungen (16, 17) gebildet sind durch eine Rastvorrichtung.

7. Sonde nach einem der Ansprüche 1 bis 6. dadurch **gekennzeichnet,** daß die Sonde eine Ansaugeinrichtung (9, 9', 9", 14) aufweist.

8. Sonde nach einem der Ansprüche 1 bis 7, dadurch **gekennzeichnet,** daß das diesseitige Ende des Rohres (8) verbunden ist mit einem Übergangsrohr (15), das mit einem Ventil (15') versehen ist.

Fig. 1

Fig. 2

Fig. 2a

Fig. 3

Fig. 4

Fig. 5

*Fig. 6*

Fig. 7a

Fig. 7b

Fig. 7c